(19) **Europäisches Patentamt / European Patent Office / Office européen des brevets**

(11) **EP 4 593 023 A1**

(12) # EUROPEAN PATENT APPLICATION

(43) Date of publication:
**30.07.2025 Bulletin 2025/31**

(21) Application number: **24382066.9**

(22) Date of filing: **25.01.2024**

(51) International Patent Classification (IPC):
**G16H 20/30** (2018.01)   **A61N 1/05** (2006.01)
**A61N 1/36** (2006.01)   **G16H 50/50** (2018.01)

(52) Cooperative Patent Classification (CPC):
**G16H 50/50; A61N 1/36038; G16H 20/30;**
A61N 1/0541; A61N 1/36039; A61N 1/36128

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Universidad de Las Palmas de Gran Canaria**
**35001 Las Palmas de Gran Canaria (ES)**

(72) Inventors:
• **HERNÁNDEZ GIL, Marcos Javier**
  **35250 Ingenio (ES)**

• **RAMOS DE MIGUEL, Ángel**
  **35412 Arucas (ES)**
• **GREINER SÁNCHEZ, David Juan**
  **35018 Las Palmas de Gran Canaria (ES)**
• **BENÍTEZ DÍAZ, Domingo**
  **35018 Las Palmas de Gran Canaria (ES)**
• **ESCOBAR SÁNCHEZ, José María**
  **35011 Las Palmas de Gran Canaria (ES)**

(74) Representative: **TRBL Intellectual Property**
**Plaza de Castilla 3, 7°A**
**28046 Madrid (ES)**

(54) **METHOD AND SYSTEM FOR OPTIMIZING MULTIPOLAR STIMULATION IN NERVE STIMULATION PROTHESES**

(57)    The present invention discloses a computer implemented method and a system for optimizing the electric current profile delivered across an array of electrodes comprised in a multipolar nerve stimulation implant. First, a finite element model of the target nerve and/or surrounding structures is built and fitted. Then, two objective functions are tailored designed to encode focusing and energy consumption, respectively. Finally, a multi-objective optimization based on evolutionary algorithms is performed over the objective functions evaluated over the FEM model, and a set of optimized electric current profiles with maximum focus and minimum energy consumption to be delivered across the array of electrodes comprised in the multipolar nerve stimulation implant is obtained.

**FIG. 1**

EP 4 593 023 A1

Description

FIELD OF THE INVENTION

The present invention belongs to the field of nerve stimulation technologies and devices in combination with advance computational models and artificial intelligence. More specifically, the invention relates to a computer implemented method for optimizing the electric current profile delivered across an array of electrodes comprised in a multipolar nerve stimulation implant. A system comprising such an implant and computing means adapted to perform the method of the invention is also presented. The present invention is particularly suitable for cochlear implants and for auditory nerve stimulation.

BACKGROUND OF THE INVENTION

Nerve stimulation or neurostimulation is the purposeful modulation of the nervous system's activity using different techniques, often within the scope of electromagnetic treatments. These consist in delivering electric and/or magnetic fields directly into the nerves or into nervous structures to stimulate the correct behavior of those that, for medical reasons, do not function correctly.

Neurostimulation can improve the life quality of those who are severely paralyzed or have profound losses to sense organs, as well as for permanent reduction of severe, chronic pain. It serves as the key part of neural prosthetics for hearing aids, such as cochlear implants, but also for artificial vision, artificial limbs, and brain-machine interfaces.

One of the most standard techniques used for neurostimulation purposes is that of the application of electric currents delivered through one or more electrodes. Depending on the target nerve or area to be treated, these electrodes can be external or can be implanted in order to maximize their efficacy, since some body regions cannot be reached appropriately by the electric currents if contact (external) electrodes are used.

A specific example of nerve stimulation implants delivering electric currents through one or more electrodes is that of cochlear implants (CI). These are surgically implanted neuro-prostheses that provide a person who has moderate-to-profound sensorineural hearing loss with sound perception by bypassing acoustic hearing using direct electrical stimulation of the auditory nerve.

Research and development in CI design has been marked by the constant search for methods to improve the efficiency and accuracy of auditory nerve stimulation. Historically, two main stimulation techniques have been used:

- Monopolar stimulation: an active electrode stimulates the auditory nerve while a neutral electrode is located outside the ear. Despite its simplicity, monopolar stimulation has limitations in targeting and can cause greater current dispersion in unwanted areas, which can result in lower efficiency and higher energy demand; and
- Multipolar stimulation: multiple active electrodes that are activated simultaneously with different current settings are used. While the multipolar technique improves targeting compared to monopolar stimulation, it still presents significant challenges in terms of energy efficiency and precise targeting.

Regarding multipolar stimulation, two types of strategies have been typically followed: electrode focusing and neural focusing. Electrode focusing tries to reduce electrode interaction from voltage spread associated with monopolar stimulation. These techniques include bipolar (BP), tripolar (TP), partial tripolar (pTP) and phased-array (PA), among others. In all of them, multipolar stimuli produce localized potential peaks that are sharper than monopolar stimuli. For the PA electrode focusing, the voltage pattern at the electrodes gives rise to a current pattern which is calculated by inverting the transimpedance matrix.

In turn, neural focusing tries to achieve a sharper peak of the voltage distribution at the target neural pathway. Several studies have searched for a focusing strategy on this direction in order to maximize the effect of the electric currents delivered by an electrode in the specific location of said electrode while minimizing their effect anywhere else. However, in all of them, focusing was achieved at the expense of more power consumption if adequate loudness levels compared to monopolar stimulation were to be reached. Moreover, none of these focusing strategies employ an optimization procedure to calculate the configuration of input currents that increase spatial selectivity of neural excitation.

Therefore, and in view of the strategies present in the state of the art, it would be necessary to provide a systematic and optimized approach to improve both the precision (focusing) of stimulation in specific areas of the target nerve (in particular of the auditory nerve) and the overall energy efficiency of the nerve stimulation implant.

SUMMARY OF THE INVENTION

To overcome the above limitations, the invention proposes a novel approach to optimize the electric current profile delivered across an array of electrodes comprised in a multipolar nerve stimulation implant. State-of-the-art techniques, such as monopolar or phase array (multipolar) approaches, are typically imbalanced in the sense that those reaching high focusing capabilities imply high energy consumption, and those with low energy or power consumption are not able to provide good focusing. The present invention provides a solution to this deficiency by presenting a method for simultaneous optimization of focusing and power/energy consumption in nerve stimulation implants.

More specifically, and advantageously, a first object of the invention relates to a computer implemented method for optimizing the electric current profile delivered across an array of electrodes comprised in a multipolar nerve stimulation implant, the method being characterized in that it comprises performing the following steps in any possible technical order:

a) provide a finite element model of the electric current density distribution in a target nerve and/or surrounding structures obtained from an electric current profile comprising a plurality of conductivity parameters;
b) fitting the values of the conductivity parameters comprised by the model provided in step a) by using real measurements of a transimpedance matrix of a plurality of subjects as input data, obtaining a fitted model;
c) providing a focus objective function that:

- is defined such that it maximizes the electric current densities reaching a plurality of neurons comprised within a predefined area centered at each electrode in the array of electrodes while minimizing the electric current densities reaching neurons corresponding to other electrodes;
- is adapted to be evaluated over the fitted model; and
- comprises first optimization parameters related to the current density profile;

d) providing a power consumption objective function that:

- is defined such that it maximizes the power consumption for a given electric current density profile delivered across the array of electrodes;
- is adapted to be evaluated over the fitted model; and
- comprises second optimization parameters related to the current density profile;

e) providing a multi-objective optimizer, based on one or more evolutionary algorithms, adapted to take as input two or more objective functions and to provide one or more sets of optimization parameters comprised by said objective functions that simultaneously optimize all the inputted objective functions;
f) executing the multi-objective optimizer provided in step e) using as input the focus objective function provided in step c) evaluated over the fitted model and the power consumption objective function provided in step d) evaluated over the fitted model, obtaining one or more sets of first and second optimization parameters that simultaneously maximize the focus objective function and minimize the power consumption objective function; and
g) deliver one or more optimized electric current profiles defined by the one or more sets of first and second optimization parameters corresponding to electric current profiles with maximum focus and minimum energy consumption to be delivered across the array of electrodes comprised in the multipolar nerve stimulation implant.

The method is then based on a methodology that uses finite element models to simulate the current distribution generated by the electrodes of the implant, whose geometry is generated from medical image data (transimpedance matrix) of the patient or others. Then, using stochastic global optimization methods such as metaheuristics, bio-inspired methods and/or evolutionary algorithms, it optimizes the current distribution generated by the implant to improve targeting in specific regions of the target nerve, while also minimizing energy consumption.

It is to be noted that separate, independent optimizations could also be performed, i.e., optimizing one of the functions disregarding the other constraint. For instance, if the main interest is to obtain the best focused current density profile, irrespectively of the associated energy consumption, only an optimization of the objective function for focusing will be carried out.

In a preferred embodiment of the invention, the nerve stimulation implant comprises a cochlear stimulation implant and wherein the target nerve comprises an auditory nerve.

In another preferred embodiment of the invention, the fitting performed in step b) comprises performing the following sub-steps:

b1) providing first sampling data of the target nerve and/or surrounding structures;
b2) provide a surrogate model of the electric current density distribution based on the sampling data provided in step b1) comprising the plurality of conductivity parameters;
b3) performing an assisted evolutionary optimization of the surrogate model by means of an evolutionary optimizer and by using real measurements of the transimpedance matrix of a plurality of subjects as input data, obtaining a first set of fitted values of the conductivity parameters; and

b4) fitting the values of the conductivity parameters comprised by the model provided in step a) by evaluating said model over the first set of fitted values obtained in step b3) and by using real measurements of the transimpedance matrix of a plurality of subjects as input data, obtaining a fitted model.

This procedure saves a considerable amount of computation time (and, therefore, a considerable amount of energy as well) since the FEM model does not need to be evaluated in every run of the fitting over the whole parameter space. It is only evaluated around a local optimal point in said parameter space of solutions for the conductivity parameters.

In another preferred embodiment of the invention, the fitting performed in step b) further includes using the location of the stimulation electrodes comprised by the electrode array after implantation and 3D models of the anatomy corresponding to the plurality of subjects as input data.

In another preferred embodiment of the invention, the electric current profiles obtained in step g) comprise at least one of the following: intensity, frequency, phase width, phase spacing and waveform values.

In another preferred embodiment of the invention, the evolutionary algorithm in which the multi-objective optimizer provided in step e) comprises a Non-dominated Sorting Genetic Algorithm II.

In another preferred embodiment of the invention, the method further comprises, after step g), performing the following step:

h) recording, using storage means, the one or more optimized electric current profiles obtained in step g).

This allows for storing the specific current patterns or profiles delivered to a patient so that they can be exactly reproduced in subsequent treatments or sessions.

A second object of the invention relates to a system comprising:

- a multipolar nerve stimulation implant comprising a plurality of electrodes arranged in an electrode array; and
- computing means, connected to the stimulation implant, and characterized in that said computing means comprise hardware and/or software means adapted to perform a method according to any of the preferred embodiments described above.

The connection between the stimulation device and the computing means may comprise a wireless connection, and the system may further comprise storage means connected to the computing means and, optionally, to the stimulation implant.

Finally, a third object of the invention relates to a computer program comprising instructions which, when the program is executed by computing means, cause the computing means to carry out a method as described above in the different embodiments.

In summary, the proposed method represents a novel approach to multipolar nerve stimulation based on obtaining the narrowest possible pattern of current densities at target neurons. Although this generally leads to higher power consumption, the proposed method allows for finding the profile of currents to be delivered by the electrodes that maximizes the focusing for a given power consumption, or alternatively equivalent, minimizes the power for a given focusing.

**BRIEF DESCRIPTION OF DRAWINGS**

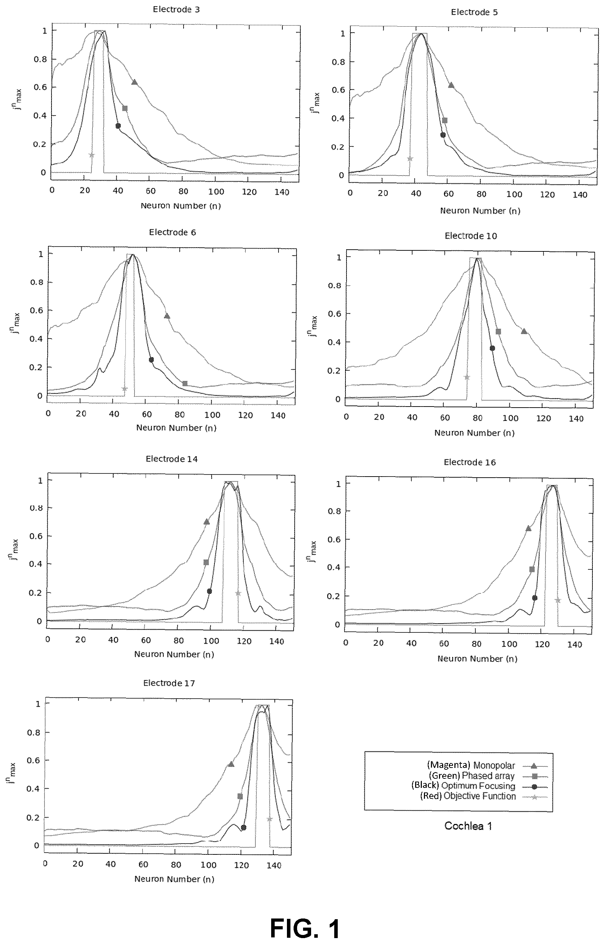

Figure 1 shows the distribution of current density $j_{max}^n$ in electrodes 3, 5, 6, 10, 14, 16 and 17 of the Cochlea 1 model for a method according to the present invention (dubbed as optimal focusing, OF), for multipolar (MP) and for phase array (PA) approaches, compared with a target objective function profile.

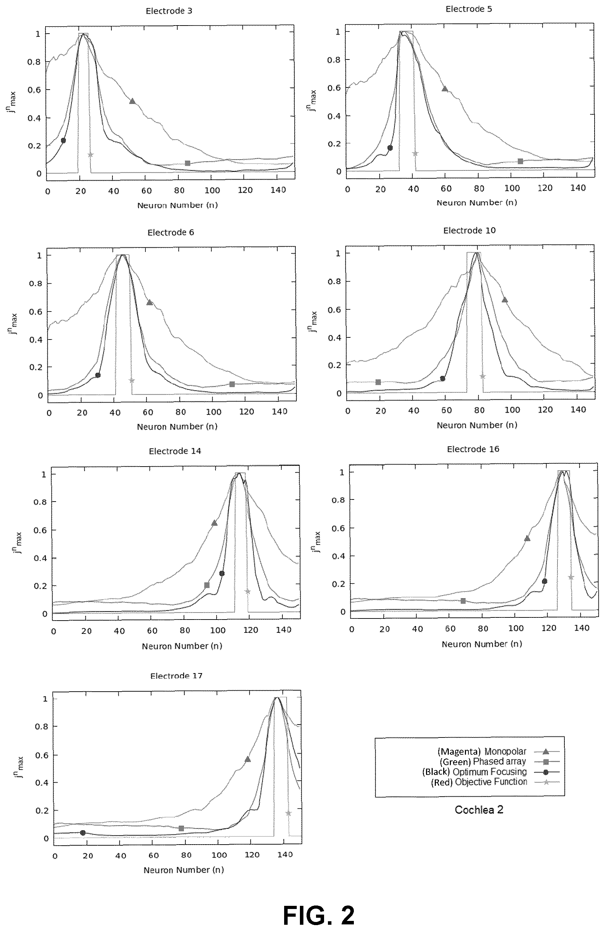

Figure 2 shows the distribution of current density $j_{max}^n$ in electrodes 3, 5, 6, 10, 14, 16 and 17 of the Cochlea 2 model for a method according to the present invention (dubbed as optimal focusing, OF), for multipolar (MP) and for phase array (PA) approaches, compared with a target objective function profile (in red).

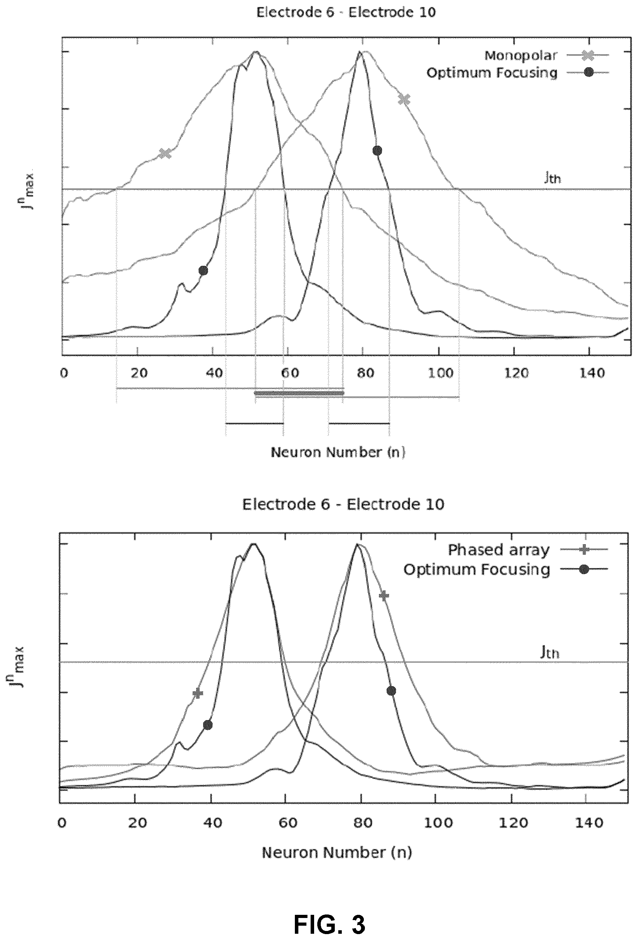

Figure 3 shows the distribution of current density $j_{max}^n$ in electrodes 6 and 10 of the Cochlea 1 model compared to that of a monopolar approach (top panel) and to that of a phase array approach (bottom panel). The cross talk between said electrodes is also displayed in the top panel.

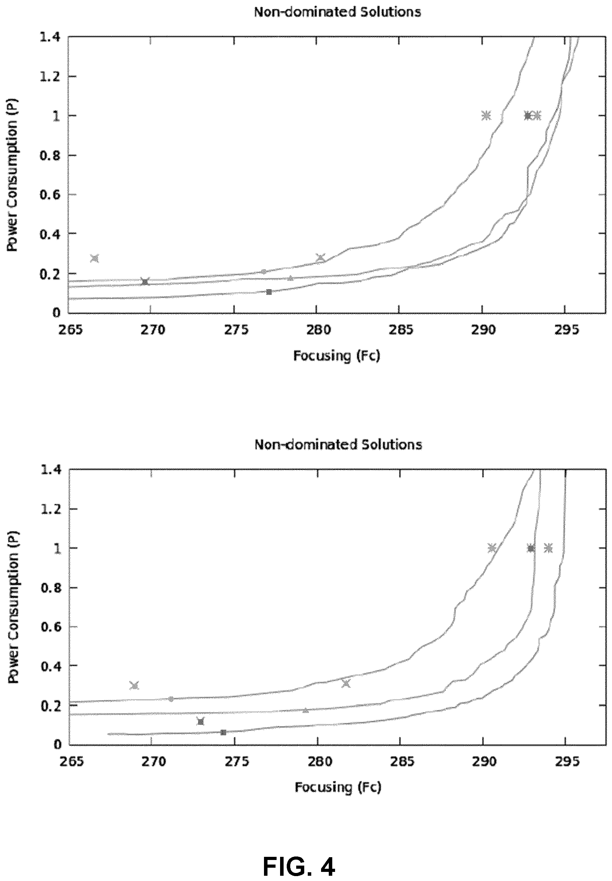

Figure 4 shows the non-dominated solutions corresponding to electrodes 3, 10, and 17 of Cochlea 1 (top panel) and Cochlea 2 (bottom panel) of the multi-objective optimization algorithm in the plane power consumption-focusing, including MP (crosses) and PA (asterisks) equivalent results.

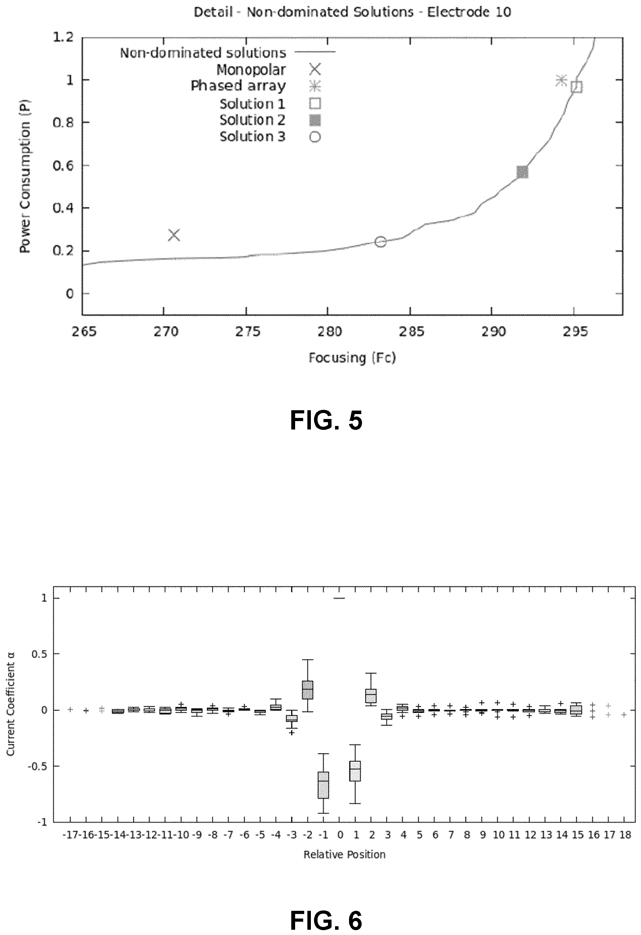

Figure 5 shows selected representative non-dominated solutions corresponding to electrode 10 of Cochlea 1 of the multi-objective optimization algorithm in the plane power consumption-focusing, including MP (crosses) and PA (asterisks) equivalent results.

Figure 6 shows the distribution of current coefficients of the solutions of the multi-objective optimization algorithm rescaled to interval [-1, 1] for electrodes 3 to 17 of Cochlea 1. Relative numbering of the electrodes has been adopted, so that the maximum coefficient is associated with relative position 0.

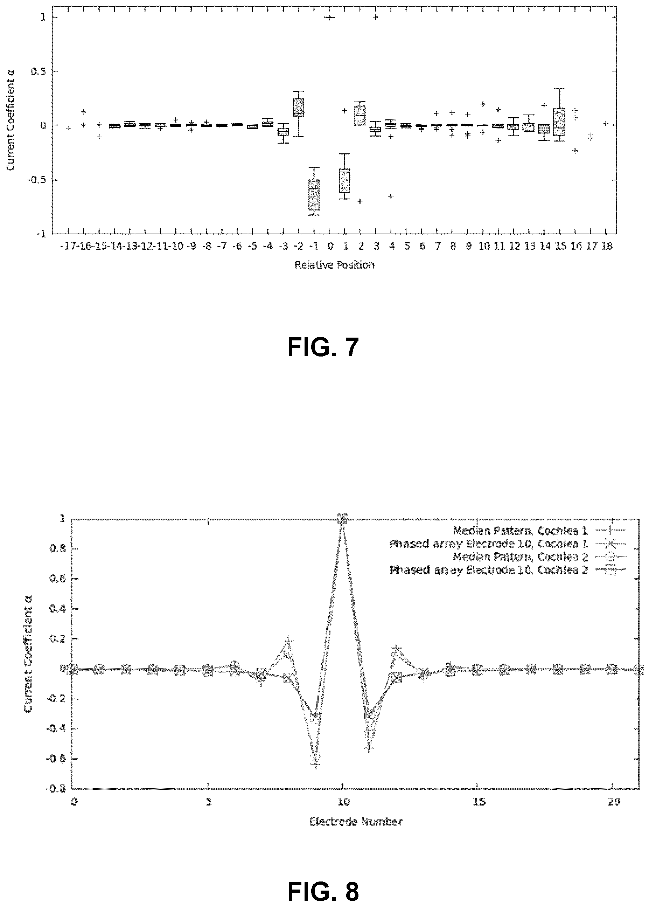

Figure 7 shows the distribution of current coefficients of the solutions of the multi-objective optimization algorithm rescaled to interval [-1, 1] for electrodes 3 to 17 of Cochlea 2. Relative numbering of the electrodes has been adopted, so that the maximum coefficient is associated with relative position 0.

Figure 8 shows the current coefficients of the solutions of the multi-objective optimization algorithm rescaled to interval [-1, 1] for electrode 10 and for the median pattern compared with PA for Cochlea 1 (top panel) and Cochlea 2 (bottom panel).

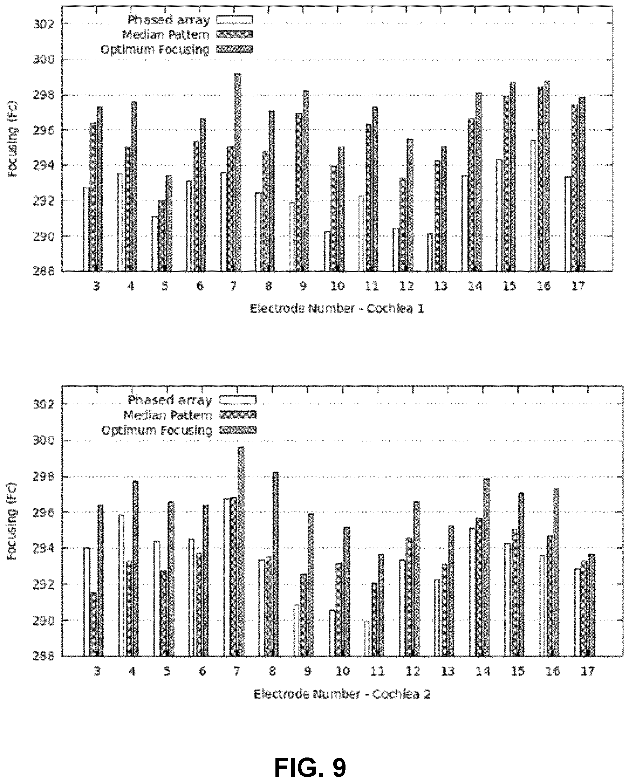

Figure 9 shows the focusing values of median pattern versus the results using the method of the invention without adopting the median pattern and versus the PA results for electrodes 3 to 17 of Cochlea 1 (top panel) and Cochlea 2 (bottom panel).

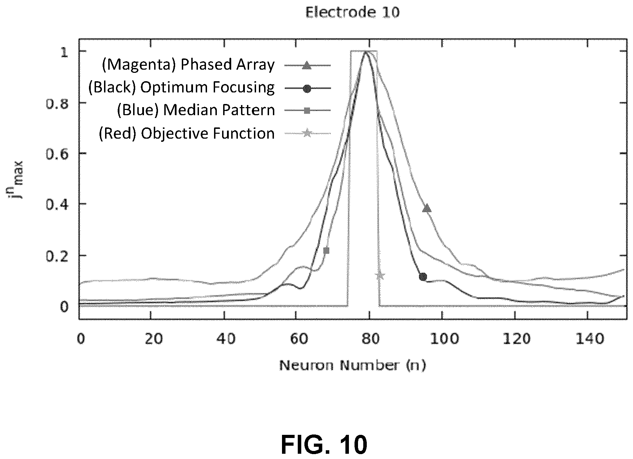

Figure 10 shows the distribution of current density $j_{max}^n$ in 10 of Cochlea 1 for a method according to the present invention (dubbed as optimal focusing, OF), for a method according to the present invention plus a median pattern, for multipolar (MP) and for phase array (PA) approaches, compared with a target objective function profile.

## DETAILED DESCRIPTION OF THE INVENTION

Some preferred embodiments of the invention, shown in Figs. 1-12, will be now described for illustrative, but not limiting purposes.

As stated, the method of the invention consists of a novel approach to multipolar (MP) stimulation at the neuronal level based on current densities reaching neurons designed to maximize focusing while minimizing energy consumption. To this end, two objective functions are proposed: one for focusing and one for power consumption, which are assessed with a finite element model (FEM) and optimized using multi-objective optimization techniques. The method of the invention therefore provides an optimized current focusing on relation to MP and phased array (PA) implant stimulation while also reducing power consumption.

An example of a realization of the invention, focused on multipolar cochlear implants (CIs) for auditory nerve stimulation, will be presented in the following for illustrative, but not limiting purposes. Multipolar cochlear implants comprise, in general, two main components:

- an outside component, typically worn behind the ear, corresponding to a sound processor containing microphones, electronics that include digital signal processors, one or more batteries, and a coil that transmits a signal to the implant across the skin, and
- an inside component comprising a coil adapted to receive signals, electronics, and an array of electrodes placed into the cochlea across which the electric currents are delivered to the auditory nerve.

The current input of a given electrode of a CI is a charge-balanced pulse train. The phase width and the interphase gap vary depending on the manufacturing company. For example, Cochlear (Cochlear Ltd. Sydney, Australia) uses, for default, a biphasic stimulus with a phase width of 25 $\mu$s, an interphase gap width of 8 $\mu$s and a frame period of 1112 $\mu$s.

Working in the frequency domain, one can write the potential at the electrodes as $V(\omega) = Z(\omega) I(\omega)$, where $Z(\omega) = (Z_{ij}(\omega))$ is the transimpedance matrix and $I(\omega)$ is the vector of the currents supplied by the electrodes (i.e., the Fourier transform of the vector of input current pulses from each electrode). The entries of the transimpedance matrix $Z_{ij}(\omega) = V_i(\omega)/I_j(\omega)$ are the relationship between the potential at the $i_{th}$ electrode $V_i$, when the $j_{th}$ electrode delivers a current $I_j$. The diagonal terms of the transimpedance matrix, $Z_{ii}(\omega)$, depend on the frequency due to the double layer interface formed between the electrolyte and the electrode, and therefore, they are complex.

Several equivalent circuits have been proposed to model these impedances which are, in essence, variations of a circuit formed by the sum of two impedances $Z_{DL} + R_T$. The first impedance, $Z_{DL}$, represents the electrolyte-electrode interface, approximated by the parallel combination of a non-faradaic pseudo capacitance $Z_{cpa} = K (j\omega)^{-\beta}$ (where $\beta$ and K are constants) and a faradaic transfer resistance $R_F$ derived from the Butler-Volmer equation. The second impedance, $R_T$,

is purely resistive and represents the sum of the resistances due to the electrolyte, tissues, and conductors.

The off-diagonal terms of $Z$ represent the potentials reached by one electrode produced by the current arising from another electrode and they are purely resistive elements. All of the above suggests that the transimpedance matrix can be written as the sum of two matrices, $Z(\omega) = D(\omega) + Z_c$, where the first matrix, $D(\omega)$, is diagonal and introduces the complex impedance, $Z_{DL}$, due to the double layer interface. The values of $Z_{DL}$ vary according to the electrode and patient under consideration. The diagonal terms of $Z_c$ represent the resistance $R_T$ of the equivalent circuit described above. The off-diagonal terms of $Z_c$ coincide with those of $Z$. For all these reasons, the matrix $Z_c$ is purely resistive (real).

The potential at the electrodes due to the current vector $I(\omega)$ is thus given by $V(\omega) = D(\omega)I(\omega) + Z_c I(\omega)$ the second term being the vector of the potentials at the electrode sites in the perilymph $V_{ph}(\omega) = Z_c I(\omega)$. The usual clinical telemetry only allows for measuring the off-diagonal inputs of $Z_c$, since they coincide with those of the transimpedance matrix $Z(\omega)$. The diagonal terms of $Z_c$ must be estimated by approximate methods through the off-diagonal entries of $Z_c$.

Performing a Fourier transform, if $i(t)$ is the input current vector at the electrodes, the corresponding potential vector, $v(t)$, is given by $v(t) = F^{-1}(Z(\omega)I(\omega)) = F^{-1}(D(\omega)I(\omega)) + Z_c F^{-1}(I(\omega)) = F^{-1}(D(\omega)I(\omega)) + Z_c i(t)$. The last term corresponds to the time-domain potential in the perilymph at the electrode positions $v_{ph}(t) = Z_c i(t)$. Thus, this potential $v_{ph}(t)$ and the intensity $i(t)$ are in phase since the matrix $Z_c$ is purely resistive. The term $F^{-1}(D(\omega)I(\omega))$ is the responsible of the distortion suffered by the potential with respect to the input $i(t)$.

When an electrode injects a current, the neurons located in its surroundings are excited. There are numerous works that study the relationship between the injected current and the extension of the region of neurons triggered by this current. They show that the threshold input current amplitude, $I_{th}$, required to activate neurons located at distance $r$ from the stimulating electrode tip is given by $I_{th} = K_e r^2$. The values of the excitability constant $K_e$ depend, among other things, on the type of neuron and the input waveform.

Each electrode can be considered as a point source of current at sufficiently large distances (on the order of several times the size of the electrode). The current density produced by a point source is given by $J = I/4\pi r^2$. Thus, it is deduced that the threshold current density $J_{th}$ is $J_{th} = K_e/4\pi$. This result shows that an action potential is elicited if the most excitable areas of a neuron, which for myelinated neurons are the nodes of Ranvier, reach a current density greater than or equal to $J_{th}$. The number of neurons that exceeds $J_{th}$ depends on the amplitude and pattern of the input currents $i(t)$.

To determine whether one current density profile is more focusing than another, the profiles are normalized. To this end, the amplitude of $i(t)$ that produces a maximum current density in the target region, $J_{max}$, is set to $J_{max} = 1$ $A/m^2$. Within this scope, in order to maximize the focusing of the stimulation implant, the aim is to find an input current pattern that results in a normalized current density distribution that is centred on the target area of the auditory nerve and that is as narrow as possible. This strategy differs from those proposed in previous works, focused on achieving focused multipolar stimulation by imposing a certain pattern of potentials on electrodes or neurons.

The first task to be performed is to build a FEM model that represents, as faithfully as possible, the potentials and current densities that appear in the cochlea when a certain channel (the set of electrodes acting simultaneously delivering the appropriate currents to stimulate a specific region of the auditory nerve) is activated. This model is not intended to simulate the double-layer phenomenon, associated with the $D(\omega)$ matrix, but to characterize the electrical behaviour that allows for calculating the potential and currents inside the cochlea, from which the conducting part of the transimpedance matrix, $Z_c$, is derived. To this end, a volume conduction model is constructed.

To accurately model the currents produced by the CI, precise knowledge of the geometry and electrical properties of all the tissues that make up the cochlea and the rest of the head is needed. This knowledge is practically impossible to obtain due to the complexity of these structures and the variability that exists between patients. To bypass this problem, a simplified conductive model is constructed such that the cochlea is included in a sphere containing a medium whose conductivity, $\sigma_{ext}$, must be adjusted so that the matrix of the model, $Z_{c,m}$, and that of the patient under consideration, $Z_{c,p}$, are as similar as possible. The model consists, in addition to $\sigma_{ext}$, of two additional parameters that must be adjusted to match both matrices: the conductivity of a layer covering the outer surface of the cochlea, $\sigma_{bone}$, which mimics the high-density bone covering the real cochlea by imposing a contact impedance condition, and the conductivity of the perilymph, $\sigma_{per}$.

The computational model of the cochlea also includes an electrode array of 22 electrodes embedded in a silicone carrier. Furthermore, due to the application of this particular example of method of the invention to auditory nerve fibers (ANFs) an approximate representation of these fibers needs to be included in the model. This is achieved by means of a set of virtual neurons (VNs) introduced as curves that imitate the trajectories of real neurons and which are used to determine the values of current densities in realistic positions. The density of real neurons in the auditory nerve is much higher than the density of VNs in this computational model, so each VN represents a large number of real neurons.

The three conductivities comprised by the model are the variables of an objective function that measures the difference between $Z_{c,m}$ and $Z_{c,p}$. The optimum conductivities are those resulting from the minimization of this objective function defined as the difference of these two matrices $\Delta_c = Z_{c,m} - Z_{c,p}$, being the specific implementation of the selected objective function the Frobenius norm of $\Delta_c = (\delta_{ij})$, $\|\Delta\_c\|_F$. Since clinical measurements do not allow for determining the diagonal terms of $Z_{c,p}$ and thus calculate $\delta_{ii}$, the calculation will be carried out taking $\delta_{ii} = 0$.

Every evaluation of the objective function involves the execution of a FEM simulation of the model to calculate $Z_{c,m}$. Therefore, an evolutionary algorithm as global optimizer with the only requirement of being able to evaluate the objective function (e.g. without any derivability condition), will be used. In order to speed-up the optimization of the evolutionary algorithm, the process will be supported with the construction of a surrogate model with much lower evaluation cost (without the need to evaluate the FEM simulation); that is, a surrogate assisted evolutionary optimization will be afforded.

First, as initial sampling, a Latin Hypercube Sampling is performed to propose the set of design variables (conductivities), including the points of full factorial approach; the objective function of those solutions is evaluated. Second, a surrogate model is built based on the previous result. Several Kriging models were tested: four types of correlation functions were taken into account: exponential, squared exponential, matern 5/2, and matern 3/2; with constant, linear or quadratic model for the deterministic term. The Kriging model with best test set accuracy was chosen after a split of the whole sampling data consisting in training set and test set. Third, once the initial surrogate model is built, a surrogate assisted evolutionary optimization is performed using differential evolution as global optimizer and the abovementioned Kriging model as surrogate. Several independent executions are run, and the best solution (consisting in the three optimized values of the conductivities) is chosen to proceed with the next step. Finally, a local search near the optimum obtained by the surrogate assisted optimization in the third step, is performed using the FEM model assessment. The minimum is chosen to build the adjusted transimpedance matrix $Z_{c,m}$ of the FEM model for each transimpedance matrix $Z_{c,p}$.

With the obtained adjusted conductivities, a conductivity model is built following the Laplace equation: $\nabla \cdot \sigma \nabla \phi = 0$ in $\Omega$, where $\phi$ is the electric potential and $\sigma$ is the conductivity. The domain $\Omega$ is formed by the cochlea and the surrounding sphere. The current density is given by $J = \sigma_n E$, with $E = -\nabla \phi$, and where $\sigma_n$ is the conductivity of the $n_{th}$ tissue or fluid, which is considered to be purely resistive (real). The potential and currents involved in the model can be considered as the amplitudes of the corresponding time signals.

Regarding the boundary conditions of the model, they are based on active and disconnected electrodes present in the electrode array for each instant. An active electrode is characterized by delivering a certain current, while a disconnected electrode is one through which no current flows, it is a floating conductor. This condition can be implemented in different ways, like, for instance, the one presented in Á. R. de Miguel, et al., "A phenomenological computational model of the evoked action potential fitted to human cochlear implant responses", PLOS Computational Biology 18 (5) (2022) e1010134. Besides this condition, the reference electrode is taken as ground $\phi = 0$, and the boundary of the domain, $\partial\Omega$, is chosen to be the surrounding sphere considered as an isolating surface $J \cdot n = 0$, so that the boundary condition is $\partial\phi/\partial n = 0$ in $\partial\Omega$.

Once the FEM model representing the potentials and current densities that appear in the cochlea when a certain channel is activated is fully characterizing, the next step of the method of the invention is to define the objective functions used to maximize the focusing and minimize the power consumed by the CI. These are the two objective functions of a multi-objective optimization process that aims to determine the set of non-dominated solutions that provide the profile of currents feeding the electrodes to achieve the maximum possible focusing for a given power consumption, or alternatively seen, the minimum power for a given focusing.

**Objective function for focusing**

Multipolar stimulation aims to minimize the current dispersion that occurs when a particular channel is activated. Each channel should concentrate the current in an area of the auditory nerve as narrow as possible. Thus, if one considers that channel k is responsible to excite the neural region close to electrode k, for each channel k, the aim is to obtain the pattern of currents supplied by the electrodes that maximize the current densities reaching the VNs near electrode k and minimize the current densities reaching any other VNs.

Let $\phi_i(x)$, $i = 1, ..., E$ be the potential in any point $x \in \Omega$ due to the current source at electrode i delivering an intensity $I_0 = 1$ A, where $E$ is the number of electrodes of the CI. Given the linearity of the Laplace equation, the normalized current density can be written as $j(x) = \sum_{i=1}^{E} \alpha_i \, j_i(x)$ where $J_i = -\sigma \Delta\phi_i$ and $\alpha_i$ are normalized coefficients with respect to the maximum possible value of $J$.

The maximum of $\|j(x)\|$ at the $n_{th}$ VN is denoted as $j_{max}^n$ and verifies $0 \leq j_{max}^n \leq 1$. The goal is to find the current coefficients $a$ such that $j_{max}^n(\alpha) = 1$ at the VNs around the electrode k (the target region) and 0 at the other VNs. More precisely, if $VN(k) = \{k_1, k_2 ..., k_M\}$ is the set of indexes of M VNs closer to electrode k than to the other electrodes, the objective function is $F(\alpha) = w \sum_{m \in VN(k)} (1 - j_{max}^n(\alpha))^2 + \sum_{m \notin VN(k)} (j_{max}^n(\alpha))^2$, where $w$ is a constant parameter whose role is to balance the weight of the two terms of the objective function.

The values of this function are included in the interval [0, $F_{max}$], being $F_{max} = N_n - M + wM$, being $N_n$ the total number of VNs and M the number of neurons belonging to VN(k). Note that the higher the values of $F(\alpha)$, the lower the focusing will be. Thus, this function gives directly encodes dispersion rather than focusing so the focusing objective function shall be defined as $F_c(\alpha) = F_{max} - F(\alpha)$ so that the maximization of $F_c(\alpha)$ implies a maximization of the focusing.

**Objective function for energy consumption**

Typically, maximizing the focus in multipolar stimulation leads to higher, inefficient energy consumption. Thus, the desire is to build and objective function that encodes this feature so that it can be minimized while maximizing the objective function defined for focusing. This is achieved by calculating, for each channel, the power associated with its current pattern.

To set a criterion for power comparison we will assume that two different configurations corresponding to the same channel must reach the same maximum current density. This requirement is achieved by working with the normalized coefficients $\alpha$.

Within the previously presented configuration, the objective function for the power consumption is defined as the ratio between the power of the configuration under study versus the power of a reference input $\alpha_0$:

$$P(\alpha) = \frac{P_\alpha}{P_{\alpha_0}} = \frac{\alpha^T Z_c \alpha}{\alpha_0^T Z_c \alpha_0}$$

The reference is taken to be a phased-array stimulation. Specifically, if $v_k = (0, \dots, 1, \dots, 0)$ is the voltage pattern of the $k_{th}$ channel of the PA, the normalized current pattern of this channel is given by $\alpha_{PA} \equiv \alpha_0 = N(J_{max}^{-1} Z_c^{-1} v_k)$, where $J_{max}$ is the maximum current density in any VN corresponding to the input $Z_c^{-1} v_k$. From now on $P_{\alpha_0}$ becomes $P_{\alpha PA}$.

This objective function measures the power consumed in the perilymph by a CI fed by a current pattern $\alpha$ compared to the power consumed by a phased-array stimulation. It is to be noted that this expression is independent of the waveform of the input; it only depends on the profile of currents feeding the electrodes and the resistive matrix $Z_c$. Additional energy loses such as those coming from the coil, processor, and other electronic components and/or from the dissipation in the double layer may be also considered.

**Multi-objective optimization**

Once the objective functions are properly defined, an optimization process needs to be applied in order to maximize the objective function for focusing while simultaneously minimizing the objective function for energy consumption. It is to be noted that separate, independent optimizations could also be performed, i.e., optimizing one of the functions disregarding the other constraint. For instance, if the main interest was to obtain the best focused current density profile, irrespectively of the associated energy consumption, only an optimization of the objective function for focusing will be carried out.

However, it is certainly advantageous to maximize the focus at the same time that the energy consumption is minimized. When optimizing two or more objective functions, a multi-objective optimization is required when they are in conflict, i.e., when improving the optimized value of one objective function is only possible if worsening the value of other optimized objective function. This is the case in the present invention, since improving the focusing is possible only when worsening power consumption (alternatively improving the value of power consumption is possible when worsening focusing). For this reason, in a multi-objective optimization procedure, instead of a single optimum solution, a set of equally optimum solutions called non-dominated solutions appear, which belong to the so-called Pareto set. In the method of the invention, the multi-objective optimization procedure aims to determine the non-dominated solutions (given each by its profile of currents to be delivered through the electrodes) to achieve the maximum possible focusing for a given power consumption, or equivalently, to achieve the minimum power consumption for a given focusing.

For the multi-objective optimization, stochastic multi-objective global optimization methods such as metaheuristics, bio-inspired methods and/or evolutionary algorithms may be used. In a preferred embodiment of the invention, the latter (evolutionary algorithms) is selected since efficient evolutionary algorithms for multi-objective optimization have been developed and applied in many real-world applications. These global optimization methods enable to obtain the whole set of non-dominated solutions in a single run of the algorithm without any other requirement to the objective function than being accurately computable, what has fostered their successful application in real-world problems.

Among the state-of-the-art multi-objective evolutionary algorithms to solve two objective optimization problems, in a preferred embodiment of the invention the Non-dominated Sorting Genetic Algorithm NSGA-II is used to simultaneously optimize the described objective functions $F_c(\alpha)$ (focusing) and $P(\alpha)$ (power consumption), maximizing focusing and minimizing power consumption.

Through this optimization a set of solutions for the optimizing parameters $\alpha$ that simultaneously maximize focusing while minimizing energy consumption are obtained. Since these parameters are directly related to the electric current densities, the current density profiles to be delivered to the electrodes comprised by the implant can be derived. This enables to deliver electric currents whose effect is maximised near each electrode site and minimised far from each electrode site, with the minimum amount of energy required.

**Efficiency of the method**

The described optimization procedure has been tested on two FEM cochlear geometries with perimodiolar insertion of the electrode array: Cochlea 1 and Cochlea 2.

First, to obtain the fitted values of the three conductivities described above Comsol Multiphysics 5.6 has been used after sampling the geometry of the cochlea with a 3D mesh composed of quadratic tetrahedral elements. The conductivities of each FEM mesh were adjusted to a patient transimpedance matrix following the procedure described.

Then, the multi-objective optimization with NSGA-II as described above was applied for each cochlear model. Standard parameters were applied: a population size of 100 individuals, 1000 generations as stopping criterion, SBX crossover probability of 1.0 with distribution index of 15, polynomial mutation with mutation rate of one divided by the number of variables of the chromosome (constituted by the 22 current coefficients $\alpha$ of each electrode of the CI) with distribution index of 20. In the following, when the current coefficients $\alpha$ are referred to, they are rescaled to the interval [-1, 1].

A single objective optimization of each fitness function (focusing and power consumption) was performed in order to obtain best individual solutions that were afterwards inserted in the initial population of the multi-objective algorithm. A differential evolution algorithm was performed five independent times choosing the best solution in each case. A population size of 110 individuals and 100 generations as stopping criterion was employed for that purpose. Within this setup, the optimal electric current density profiles were obtained for the implants corresponding to each cochlea model. The results for the so-called optimum focusing (OF) case, i.e., the one obtained by applying the method of the invention, were compared with the equivalent phased-array (PA) and monopolar (MP) stimulations.

Figures 1 and 2 represent maximum current densities $j_{max}^n$. They include seven representative electrodes out of the total of 22 in Cochlea 1 and Cochlea 2, respectively. The value of M of these electrodes varies from 5 to 10, being M the cardinal of the closer VNs to electrode k than to the other electrodes. A total of $N_n$ equal to 151 VNs were distributed throughout the cochlea in each test case as shown in the abscissa axis of the figures. It is clearly appreciable how the optimum focusing solutions (black lines) are able to fit properly towards the objective functions (red lines), being those optimum profiles narrower than phased-array stimulation (green lines) and them narrower than the monopolar (magenta lines) in all cases. In all cases focusing and power consumption increase from monopolar to multipolar and from multipolar to optimum focusing solutions. From all these results, it has been evidenced the capability of the methodology to improve phased-array stimulation in CI for maximizing the focusing while reducing the energy consumption.

Furthermore, due to the optimized focusing, higher narrowness is achieved for OF with respect to other methods, as shown in Figures 1 and 2. This also reduces crosstalk between Channels (i.e., interaction and distortions) as depicted in Figure 3 representing electrodes 6 and 10 in Cochlea 1 compared with the monopolar approach (top panel) and with the phased-array approach (bottom panel).

As further support for the effectiveness of the method of the invention, Figure 4 is presented. There, a set of non-dominated solutions represented as lines in the power consumption-focusing plane is presented for three chosen electrodes (3, 10, and 17) in Cochlea 1 (top panel) and Cochlea 2 (bottom panel). Each line thus represents the values of maximum focusing for each value of power consumption or, alternatively, the values of minimum power consumption for each value of focusing. A comparison with the monopolar and standard multipolar cases is also included as crosses and asterisks (with a circle, square or triangle overlay to differentiate between the different solutions), respectively. Clearly, there are non-dominated solutions that improve simultaneously focusing and power consumption in each case.

As an example, Figure 5 shows the case of electrode 10 of Cochlea 1. Three representative non-dominated solutions named Solution 1, 2 and 3 were selected. Solution 1 improves the phased-array stimulation in both objective functions, solution 3 improves the monopolar solution in both objective functions, and solution 2 attains almost the focusing of the phased-array stimulation with only half of the power consumption.

Beyond the presented results, an analysis of optimum attained solutions can lead to interesting design principles. With this purpose for focusing maximization, the optimum values of the current coefficients $\alpha$ of each electrode from 3 to 17 have been superimposed in Figures 6 and 7, respectively for Cochlea 1 and Cochlea 2. In each case, the relative position 0 belongs to each central active electrode. As observed in both figures, a clear shared pattern emerges that enables focusing maximization: the previous and posterior adjacent values (relative positions -1 and +1, respectively) with respect to the central active electrode (relative position 0) are negative and smaller; the next adjacent values (relative positions -2 and +2, respectively) are positive and again smaller than the directly adjacent values; and so on, alternating signs and continuing in the decrement of the values of the coefficients. As relevant shared values of the pattern in Cochlea 1 and Cochlea 2 the median of the distribution of the values belonging to relative positions: -4, -3, -2, -1, 0, +1, +2, +3, +4 is chosen. Distribution of current coefficients of this median pattern common to all electrodes versus those of the phased-array stimulation (e.g. of Electrode 10) of both test cases are shown in Figure 8. A clear difference emerges between both configurations, as in the phased-array stimulation only negative values of the coefficients remain, without sign oscillation.

If using those pattern values as current stimulation in the CI, Figure 9 shows the focusing for the different electrode numbers for Cochlea 1 (top panel) and Cochlea 2 (bottom panel) for monopolar, phased array, and optimum focusing approaches. It can be seen that the focusing slightly diminishes with respect to the optimum solution. However, this median pattern is able to improve the focusing of the phased-array stimulation solutions consistently in both test cases Cochlea 1 and Cochlea 2, with the only exception of electrodes 3, 4, 5 and 6 of Cochlea 2. As seen in relative position +15 of Figure 7, the dispersion of solutions in this position is larger in Cochlea 2 than in Cochlea 1. These values belong to electrodes 3, 4, 5 and 6 of Cochlea 2, whose larger variability decreases the focusing of the median pattern in Cochlea 2. Therefore, from these results, the pattern of current coefficients is more reliable when their boxplots in the extreme ranges remain with low dispersion.

The direct relationship between the focusing and the narrowness of the profile of the current densities $j_{max}^n$ along the VNs, as shown previously in Figures 1 and 2, can be observed in Figure 10. When comparing PA stimulation (magenta), median pattern (blue) and optimum focusing (black) curves, the median pattern clearly outperforms phased-array stimulation.

All these results evidence that the method of the invention provides an efficient procedure for optimizing the electric current profile delivered across an array of electrodes comprised in a multipolar nerve stimulation implant. Specifically, a computational model that combines FEM and evolutionary algorithms to simultaneously optimize focusing and power consumption in CI stimulation has been presented. Using multi-objective optimization over two designed tailored-designed objective functions, current profiles capable of improving the focusing and power consumption of both, monopolar and phased-array stimulation have been obtained.

**Claims**

1.   Computer implemented method for optimizing the electric current profile delivered across an array of electrodes comprised in a multipolar nerve stimulation implant, the method being **characterized in that** it comprises performing the following steps in any possible technical order:

a) provide a finite element model of the electric current density distribution in a target nerve and/or surrounding structures obtained from an electric current profile comprising a plurality of conductivity parameters;
b) fitting the values of the conductivity parameters comprised by the model provided in step a) by using real measurements of a transimpedance matrix of a plurality of subjects as input data, obtaining a fitted model;
c) providing a focus objective function that:

- is defined such that it maximizes the electric current densities reaching a plurality of neurons comprised within a predefined area centered at each electrode in the array of electrodes while minimizing the electric current densities reaching neurons corresponding to other electrodes;
- is adapted to be evaluated over the fitted model; and
- comprises first optimization parameters related to the current density profile;

d) providing a power consumption objective function that:

- is defined such that it maximizes the power consumption for a given electric current density profile delivered across the array of electrodes;
- is adapted to be evaluated over the fitted model; and
- comprises second optimization parameters related to the current density profile;

e) providing a multi-objective optimizer, based on one or more evolutionary algorithms, adapted to take as input two or more objective functions and to provide one or more sets of optimization parameters comprised by said objective functions that simultaneously optimize all the inputted objective functions;

f) executing the multi-objective optimizer provided in step e) using as input the focus objective function provided in step c) evaluated over the fitted model and the power consumption objective function provided in step d) evaluated over the fitted model, obtaining one or more sets of first and second optimization parameters that simultaneously maximize the focus objective function and minimize the power consumption objective function; and

g) deliver one or more optimized electric current profiles defined by the one or more sets of first and second optimization parameters corresponding to electric current profiles with maximum focus and minimum energy consumption to be delivered across the array of electrodes comprised in the multipolar nerve stimulation implant.

2. Method according to the preceding claim, wherein the nerve stimulation implant comprises a cochlear stimulation implant and wherein the target nerve comprises an auditory nerve.

3. Method according to any of the preceding claims, wherein the fitting performed in step b) comprises performing the following sub-steps:

b1) providing first sampling data of the target nerve and/or surrounding structures;
b2) provide a surrogate model of the electric current density distribution based on the sampling data provided in step b1) comprising the plurality of conductivity parameters;
b3) performing an assisted evolutionary optimization of the surrogate model by means of an evolutionary optimizer and by using real measurements of the transimpedance matrix of a plurality of subjects as input data, obtaining a first set of fitted values of the conductivity parameters; and
b4) fitting the values of the conductivity parameters comprised by the model provided in step a) by evaluating said model over the first set of fitted values obtained in step b3) and by using real measurements of the transimpedance matrix of a plurality of subjects as input data, obtaining a fitted model.

4. Method according to any of the preceding claims, wherein the fitting performed in step b) further includes using the location of the stimulation electrodes comprised by the electrode array after implantation and 3D models of the anatomy corresponding to the plurality of subjects as input data.

5. Method according to any of the preceding claims, wherein the electric current profiles obtained in step g) comprise at least one of the following: intensity, frequency, phase width, phase spacing and waveform values.

6. Method according to any of the preceding claims, wherein the evolutionary algorithm in which the multi-objective optimizer provided in step e) comprises a Non-dominated Sorting Genetic Algorithm II.

7. Method according to any of the preceding claims, further comprising, after step g), performing the following step:
h) recording, using storage means, the one or more optimized electric current profiles obtained in step g).

8. System comprising:

- a multipolar nerve stimulation implant comprising a plurality of electrodes arranged in an electrode array; and
- computing means, connected to the stimulation implant, and **characterized in that** said computing means comprise hardware and/or software means adapted to perform a method according to any of claims 1-6.

9. System according to the preceding claim, further comprising storage means connected to the computing means and, optionally, to the stimulation implant, adapted to perform a method according to claim 7.

10. System according to any of claims 8 or 9, wherein the connection between the stimulation device and the computing means comprises a wireless connection.

11. Computer program comprising instructions which, when the program is executed by computing means, cause the computing means to carry out a method according to any of claims 1-7.

FIG. 1

Electrode 3

Electrode 5

Electrode 6

Electrode 10

Electrode 14

Electrode 16

Electrode 17

(Magenta) Monopolar
(Green) Phased array
(Black) Optimum Focusing
(Red) Objective Function

Cochlea 2

# FIG. 2

Electrode 6 - Electrode 10

Electrode 6 - Electrode 10

FIG. 3

FIG. 4

**FIG. 5**

**FIG. 6**

# FIG. 7

# FIG. 8

FIG. 9

Electrode 10

FIG. 10

Europäisches
Patentamt

European
Patent Office

Office européen
des brevets

# EUROPEAN SEARCH REPORT

**Application Number**

EP 24 38 2066

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ÁNGEL RAMOS-DE-MIGUEL ET AL: "A multiobjective optimization procedure for the electrode design of cochlear implants", INTERNATIONAL JOURNAL FOR NUMERICAL METHODS IN BIOMEDICAL ENGINEERING, JOHN WILEY & SONS, INC, HOBOKEN, USA, vol. 34, no. 8, 17 May 2018 (2018-05-17), page n/a, XP072453312, ISSN: 2040-7939, DOI: 10.1002/CNM.2992 * abstract; sections 2 and 3 * | 1-11 | INV. G16H20/30 A61N1/05 A61N1/36 G16H50/50 |
| A | CHEN Y-B ET AL: "Optimization of Cochlear Implant Electrode Array Using Genetic Algorithms and Computational Neuroscience Models", IEEE TRANSACTIONS ON MAGNETICS, IEEE, USA, vol. 40, no. 2, 31 March 2004 (2004-03-31), pages 639-642, XP011110633, ISSN: 0018-9464, DOI: 10.1109/TMAG.2004.824912 * the whole document * | 1-11 | |
| A | BONHAM B H ET AL: "Current focusing and steering: Modeling, physiology, and psychophysics", HEARING RESEARCH, ELSEVIER SCIENCE PUBLISHERS , AMSTERDAM, NL, vol. 242, no. 1-2, 6 April 2008 (2008-04-06), pages 141-153, XP023610896, ISSN: 0378-5955, DOI: 10.1016/J.HEARES.2008.03.006 [retrieved on 2008-04-06] * the whole document * | 1-11 | **TECHNICAL FIELDS SEARCHED (IPC)** <br><br> G16H <br> A61N |

-/--

The present search report has been drawn up for all claims

| Place of search | Date of completion of the search | Examiner |
|---|---|---|
| Munich | 17 June 2024 | Heidrich, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 24 38 2066

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EDGAR PEÑA ET AL: "Particle swarm optimization for programming deep brain stimulation arrays", JOURNAL OF NEURAL ENGINEERING, INSTITUTE OF PHYSICS PUBLISHING, BRISTOL, GB, vol. 14, no. 1, 9 January 2017 (2017-01-09), page 16014, XP020312955, ISSN: 1741-2552, DOI: 10.1088/1741-2552/AA52D1 [retrieved on 2017-01-09] * the whole document * ----- | 1-11 | |

TECHNICAL FIELDS
SEARCHED     (IPC)

The present search report has been drawn up for all claims

| Place of search | Date of completion of the search | Examiner |
|---|---|---|
| Munich | 17 June 2024 | Heidrich, Alexander |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Á. R. DE MIGUEL et al.** A phenomenological computational model of the evoked action potential fitted to human cochlear implant responses. *PLOS Computational Biology*, 2022, vol. 18 (5), e1010134